# EUROPEAN PATENT SPECIFICATION

(11) **EP 2 295 106 B1**
(45) Date of publication and mention of the grant of the patent: **05.08.2020**
(21) Application number: 10012593.9
(22) Date of filing: 29.06.2001
(51) Int. Cl.: A61M 16/16, A61M 16/10, A61M 13/00

(54) **Apparatus for humidification and warming of air**
Vorrichtung zum Befeuchten und Erwärmen von Luft
Dispositif pour chauffer et humidifer de l'air

(30) Priority: 30.06.2000 US 215442 P
(43) Date of publication of application: 16.03.2011
(62) Divisional of application: 01947705.8
(73) Proprietor: Northgate Technologies Inc., Elgin, IL 60123 (US)
(72) Inventor: Mantell, Robert A., Arlington Heights, IL 60004 (US); Manzie, Peter A., Burwyn, IL 60402 (US)
(74) Representative: Grünecker Patent- und Rechtsanwälte PartG mbB

(56) References cited:
- EP-A- 0 567 158
- EP-A- 1 005 878
- US-A- 3 659 604
- US-A- 4 054 622
- US-A- 4 652 408
- US-A- 5 062 145
- US-A- 5 179 966
- US-A- 6 068 609

## Description

Applicants claim, under 35 U.S.C. § 119(e), the benefit of priority of the filing date of June 30, 2000 of United States Provisional Patent Application Serial No. 60/215,442 , filed on the aforementioned date.

### BACKGROUND OF THE INVENTION Field of the Invention

The present invention relates to an apparatus used to humidify and/or warm a gas prior to its use in a surgical or other medical procedure.

Table of conversion:
100 degrees Fahrenheit = 37,77 degrees Celsius.
1 inch = 2,54 cm.

### Discussion of Related Art

Many medical and surgical procedures require the supply to a patient of warmed and/or humidified gas at constant high flow rates. Ideally, the flow rate should be approximately 20 liters per minute, the relative humidity should be approximately 80 to 100 percent, and the temperature approximately 90 to 105 degrees Fahrenheit. Most prior art devices cannot meet or exceed these ideal characteristics. The flow rate of many prior devices is well below 20 liters per minute. Commonly, the flow rate of prior devices has been generally 12 to 14 liters per minute. Most of these devices generally operate by forcing the gas through the humidification material, thereby requiring a high degree of pressure. This increased pressure reduces the flow rate of the gas even further.

Document US 6 068 609 A relates to an apparatus for conditioning gas for use in a medical procedure, such as endoscopy, the gas being received into the apparatus from a gas source. The apparatus comprises a housing defining a chamber having an entry port and an exit port. A humidification means comprising at least one water-retainer layer is disposed within the chamber in the path of travel of the gas for humidifying the gas as it passes through the chamber. A humidity sensor is disposed within the chamber that senses the humidity of the gas exiting the chamber. A monitoring circuit is connected to the humidity sensor that detects when the chamber requires a recharge of liquid based on the humidity of the gas in the chamber, and generates a recharge signal indicative thereof. A charging port on the housing provides access into the chamber to recharge the chamber with water. A heating element and temperature sensor are also disposed within the chamber. A control circuit further regulates the temperature of the gas exiting the chamber.

Document EP 0 567 158 A relates to a humidifier (1) is disclosed for providing humidified gases to a patient. The humidifier (1) is very compact, having a gases volume of the order of 60mls. A microporous water compartment (16) is provided perpendicular to the gases flow (37, 78, 80) to ensure efficient humidification of the gases. Also, a combined temperature probe and heater supply connector (26) is provided which only supplies energy to the heater (22) in the water compartment (16) when the gases temperature sensor (34) is appropriately positioned in the humidifier gases passageway (2). This reduces the risk of gases at excessively high temperatures being supplied to the patient.

Document US 5 062 145 A relates to a humidifier of small gases passageway volume (of the order of 50 ml gases space) has a heater within a water containing envelope having a microporous wall common to the water space within the envelope and the gases space, the microporous wall being permeable to water vapour but not liquid water and the envelope being reinforced by a support which also directs the flow of gases over the surface of the envelope. The heater is wound as a flat spiral to avoid the effect of gases or water vapour bubbles on heater performance temperature.

Document US 3 659 604 A relates to a humidifying means has a container for water which is removable from a source of heat, with the container being provided with a cover defining an air space with an inlet for gases and an outlet for gases and water vapor, the whole being removable for sterilization or other purposes. Temperature control means are provided externally of the container to control the temperature of the water in the container. A hose connects the outlet for gases and water vapor to a patient undergoing assisted or artificial respiration and this hose has a heating element therein arranged longitudinally to reduce or obviate condensation in the hose.

Document US 4 652 408 A relates to a humidifier includes a chamber 14 having an inlet 30 and an outlet 6. The chamber 14 is releasably attached to a heater unit including a heater 22 and a heat sensor 24. When the chamber 14 and the heater unit are assembled together the heater 22 is in thermal contact with a heat transfer surface of the chamber 14 at or adjacent the inlet port 30 while the temperature sensor 24 is in thermal contact with the heat transfer surface at or adjacent the outlet port 6.

Document US 5 179 966 A relates to an article is provided in which a flavor generating medium is electrically heated to combustion to evolve inhalable flavors or other components in vapor and/or aerosol form. The article has a plurality of charges of the flavor generating medium which are heated to combustion sequentially to provide individual puffs.

### SUMMARY OF INVENTION

The invention is defined by the appended claims.

One aspect of the present invention regards a gas humidification apparatus is for the use in a medical procedure comprising: a housing (226) defining a channel having a gas inlet (212) and a gas outlet (228) in fluid communication with a humidification device; said humidification device being in fluid communication with said inlet, said humidification device comprising: a heater (18) for warming a gas flowing through the channel; a humidification material (224) adjacent to said heater (18) that readily absorbs moisture and readily releases moisture when exposed to a dry environment; an insufflator (10) comprising a gas source and connected to said humidification device receiving said gas from said insufflator which receives said gas from said gas source characterized in that a control (100) is tied to said insufflator so as to control said heater and said insufflator by dynamically controlling temperature, gas volume, gas flow rate and humidity.

The above aspect provides the advantage of supplying a patient with warmed and/or humidified gas at or near preferred rates, humidity and/or temperature.

The foregoing and other features and advantages of the invention will become further apparent from the following detailed description of the presently preferred embodiments, read in conjunction with the accompanying drawings.

### BRIEF DESCRIPTION OF SEVERAL VIEWS OF THE DRAWINGS

Figure 1 shows an example of a gas warmer and/or humidifier apparatus according to the present invention;
Figure 2 shows an example of a gas warmer and/or humidifier apparatus not forming part of the present invention having a plurality of baffles in the shell;
Figure 3 shows an example of a gas warmer and/or humidifier apparatus according to the present invention having an external temperature or humidity sensor;
Figure 4 shows a cross section perspective view of a gas warmer and/or humidifier apparatus;
Figure 5 shows a perspective exploded view of a first embodiment of gas humidification apparatus according to the present invention;
Figure 6 shows a right top side perspective view of the gas humidification apparatus of Figure 5;
Figure 7 shows a right bottom side perspective view of the gas humidification apparatus of Figure 5;
Figure 8 shows a top view of the gas humidification apparatus of Figure 5;
Figure 9 shows a right side view of the gas humidification apparatus of Figure 5;
Figure 10 shows a front view of the gas humidification apparatus of Figure 5;
Figure 11 shows a rear side perspective view of the gas humidification apparatus of Figure 5;
Figure 12 shows a top view of an embodiment of a humidification material to be used with the gas humidification apparatus of Figure 5;
Figure 13 shows a perspective view of a second embodiment of gas humidification apparatus according to the present Invention;
Figure 14 shows a side view of the gas humidification apparatus of Figure 13;
Figure 15 shows a partially exposed side view of the gas humidification apparatus of Figure 13;
Figure 16 shows a right front side and partially exposed perspective view of the gas humidification apparatus of Figure 13;
Figure 17 shows a right rear side and partially exposed perspective view of the gas humidification apparatus of Figure 13; and
Figure 18 shows a circuit diagram of heating circuit that can be used with the gas humidification apparatus/gas warmer and/or heater apparati of Figures 1-17.

### DETAILED DESCRIPTION OF THE PREFERRED EMBODIMENTS

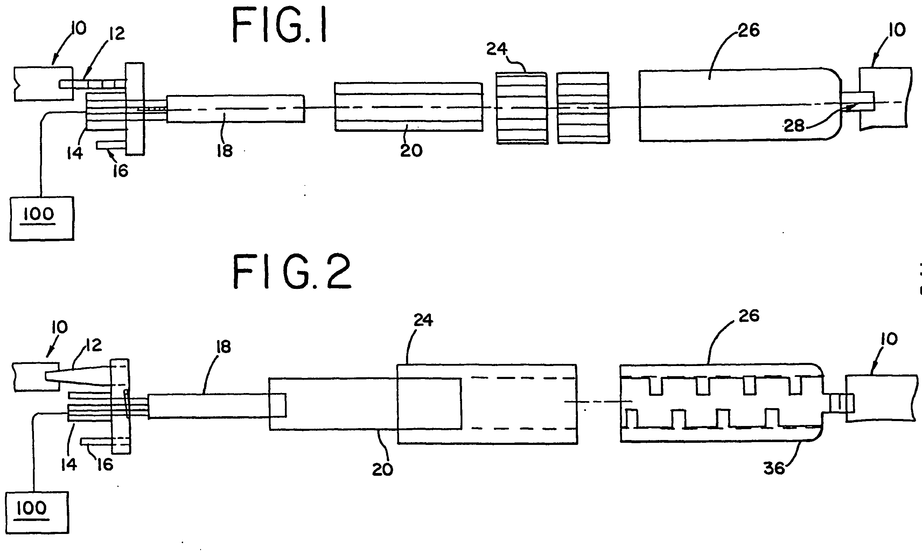

Figure 1 shows an example of the gas warmer and humidification apparatus not according to the invention. Figure 1 shows the apparatus used in conjunction with an insufflation device. Figs. 1-3 show the apparatus 1 associated with the insufflation tubing 10. The apparatus is located downstream from the gas source for the insufflation device where downstream refers to a location closer to output of the insufflation tubing 10 or a patient. The apparatus 1 has an upstream end located nearer to the gas source and a downstream end located closer to the patient. The gas warmer and humidifier apparatus 1 may be constructed as a re-useable or disposable product.

As shown In Fig. 1, a gas Inlet port 12 is located at an upstream end of the apparatus 1 and associable with the insufflation tubing 10. A plurality of plugs 14 may also be located at the upstream end of the apparatus 1. The plugs 14 may be male leads for association with the heater 18 and/or a thermocouple and/or other suitable sensing devices. It is to be understood that the location at the upstream end is variable and other locations consistent with the characteristics of the plugs 14 are envisioned.

As shown in Figs. 1-3, the general arrangement of the apparatus 1 follows. The apparatus 1 includes a heater 18. Surrounding the heater 18 is a core 20. The core 20 maintains the heater 18 in a significantly watertight environment. About the core 20 is a humidification material 24. The humidification material 24 generally envelops the entire core 20. The humidification material 24 may only partially envelop the core 20 as well. A shell 26, acting as a housing, surrounds humidification material 24. At the downstream end of the apparatus 1 may be a gas outlet 28 associable with a downstream portion of the insufflation tube 10.

The heater 18 may include a conventional cartridge heater, a heat generating wire, a light bulb, or other heat generating device capable of creating an elevated temperature that can radiate from the surface of the heater. As shown in Figure 1, the heater 18 is insertable within a core 20 of non-conductive material. As shown in Fig. 2, the heater 18 and plugs 14 are molded into a single assembly that is then molded with the core 20 to make a single unit.

The heater 18 can be a metal structure with integral sensing elements or eternal sensing elements. It can also be molded of a high temperature resistant plastic. Either the metal or the plastic heater 18 is disposable, although the lower cost of the plastic heater 18 may better suit it as a disposable heater 18. Further, the disposability or re-usability of the apparatus 1 aids in maintaining the apparatus 1 sterile for any purposes that may require a sterile apparatus 1.

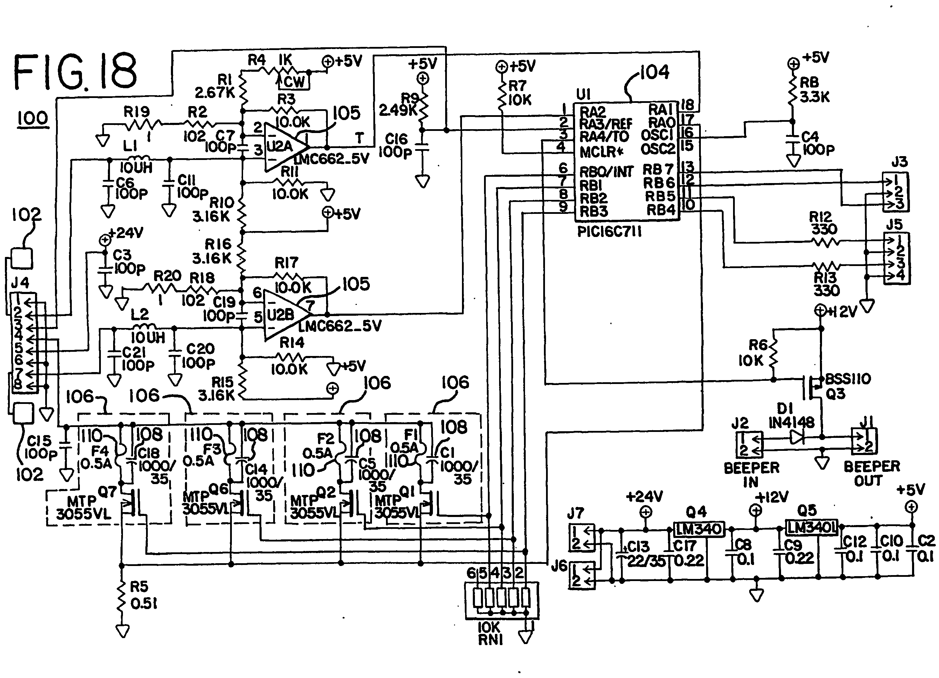

The heater 18 has approximately 36 watts of power although heaters 18 with other wattage, such as between 10 watts and 50 watts, can also be used. The heater 18 typically is approximately 1 to 5 inches long, preferably approximately 1½ to 3 inches long, but other sizes can be used depending on the physical size of the other components, and the amount of humidity to be generated. As shown in FIGS. 1-4 and 18, the heater 18 may be connected to control circuitry 100 controls the amount of heat and rate of heat generated by the heater 18. As shown in FIG. 18, the control circuitry 100 includes one or more temperature sensors 102 and a control system 104 to regulate the degree of energy supplied to the heater 18 by modulating the current supplied to the heater via turning on/off the current and raising or lowering the current. In the case of using two temperature sensors 102, the temperature sensors 102 each independently measure the temperature of the core 20. The temperature signals from temperature sensors 102 are continuously fed to amplifiers 105. The two signals are compared with each other and if it is determined that the difference between the signals reaches or exceeds a predetermined level, such as 5°C, then the control system 104 turns off the current drivers 106 and the current supplied to the heater 18. The current drivers 106 are turned off because reaching or exceeding the predetermined level denotes that one or both of the sensors 102 are defective and need to be replaced.

Assuming that the sensors 102 are not deemed defective, the control system 104 includes four identical current drivers 106 that are in parallel with one another as shown in FIG. 18. Each driver 106 provides an output that is identical with the outputs of the other three drivers 106. The control system 104 will drive each of the outputs of the current drivers 106 with approximately a 25% duty cycle wave shape. The four drivers combined will provide approximately 100% drive to the heater 18. Each driver 106 includes a capacitor 108 of 1000µ F in parallel with a fuse 110. The capacitors 108 direct the current during its respective 25% duty cycle away from its corresponding fuse 110. In the event that a single driver 106 fails, allowing continuous current flow, the corresponding capacitor 108 will charge up and allow current to flow through the corresponding fuse 110. In less than approximately 2 seconds, the fuse 110 in the driver circuit 106 will create an open circuit, thus preventing uncontrolled current to flow to the heater 18.

The apparatus 1 can have wiring to the heater 18 permanently attached. In Fig. 1, the apparatus 1 can have wiring to the heater 18 constructed with an Integral connector that can be molded into the apparatus 1 or connected/disconnected via a one time use tab connection system. The apparatus 1 can have wiring to the heater 18 with the terminations molded into a natural connector, so that the cabling can be plugged into it, reducing its cost. The electronic wiring used to provide power and to measure the temperature or humidity can be wired directly to the active elements and over molded. The output wires will be molded or inserted into the shell 26 in order to make the cord detachable from the apparatus 1.

The heater 18 may be controlled by conventional heater controllers as are available on the market, such as those made by Watlow. Controllers typically are designed to work with temperature sensing devices such as thermocouples resistance temperature detectors (RTD's) and or thermistors.

Optionally, the apparatus 1 can be provided with additional circuitry to measure humidity using a humidity sensor. Humidity sensors are available through Omega Engineering located in Atlanta, Georgia, which can supply both the sensor and circuitry for reading and display. Additionally, optionally, the temperature of the gas and the humidity of the gas could be displayed with additional circuitry. A remote power unit, part of the insufflator, or part of any other device used in the Operating Room associated with endoscopic procedures could provide the additional circuitry to display this information. Based on the readings, adjustments could be made on the amount of moisture fed to the humidification material 24, or how much heat should be applied, or both.

A control could also be tied to the Insufflator to supply the circuitry mentioned above. By monitoring characteristics in temperature, gas volume used, gas flow rate and/or humidity readings, the insufflator could dynamically control the variables to maintain optimum conditions.

The core 20 may be made of, but not limited to, plastic or a sheet metal. Some of the plastics that may be used for the core 20 include polycarbonate, Ryton™, Vesper™, or any of the high temperature plastics. A sheet metal such as aluminum coated with a non-conductive substance may also be used for the core 20.

As shown in Figure 1, the apparatus 1 includes a humidification material 24. The humidification material 24 both readily absorbs moisture and readily releases it when exposed to a dry environment. Materials such as nylon and cotton are just a few of the many commercially available fibers that can meet these requirements. The humidification material 24 can have a tubular inside and outside surface. Tubular refers to a smooth surface. Yet, it is envisioned that the humidification material 24 may have a patterned or varying 15 degrees of a non-smooth surface.

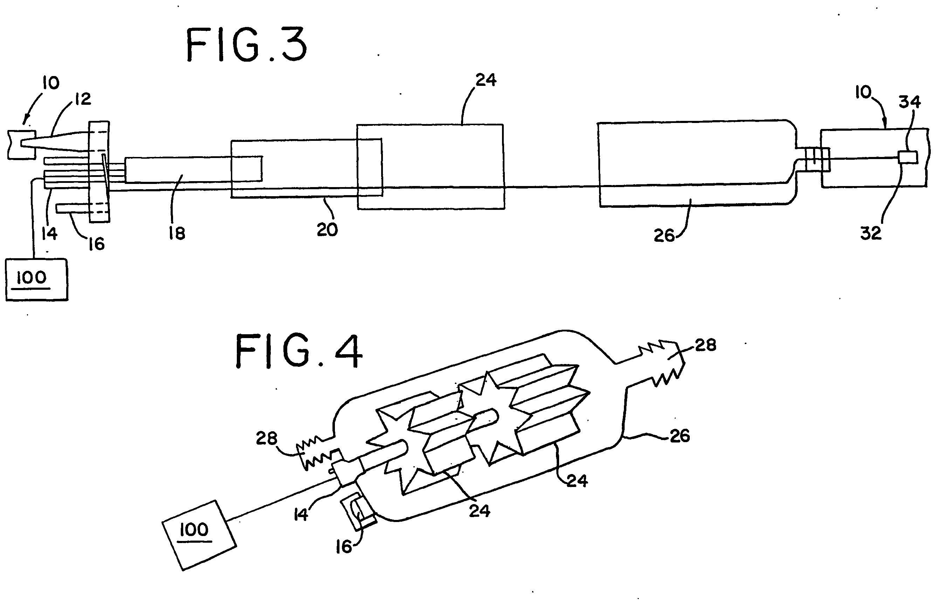

As shown in Fig. 4 , the humidification material 24 used has a smooth inner surface and a serrated or star-like shaped outer surface to maximize surface area in the shortest possible linear space. Fig. 4 shows the example including a first and a second section of the humidification material 24. Each section of the humidification material 24 is approximately an inch long with an inner channel in intimate contact with the heater 18. Each of these serrated sections is slid over the core 20 that contains the heater 18. Preferably, a ¼ Inch gap should be between the serrated sections. A plastic spacer may be inserted between the serrated sections to provide the gap. The first and second serrated sections should be set out of phase with each other to force turbulence of the gas and increase the surface area of the material as it passes over the sections. Note that the first and second serrated sections can be formed from a single serrated material by cutting the single serrated material so that the two serrated sections are formed. After cutting, the two serrated sections are rotated relative to one another until the desired phase difference between the two sections is achieved.

The flow of CO₂ gas over the absorbent material is affected by the shape of the absorbent material and/or the channel within the shell 26. The absorbent humidification material 24 may be cylindrically shaped and surrounded by a coil used to direct the flow of CO₂ gas. As the CO₂ gas travels through the windings of the coil, warmth and humidity are transferred to the CO₂ gas. The external surfaces of the coil rest against the inside of the shell 26 forming a seal that forces the CO₂ gas to travel through or within the coil windings.

Other shapes and sizes can be used for the humidification material 24. Manufacturers of this humidification material 24 are Pall Medical located In East Hills, New York and Filtrona Richmond Inc. located in Richmond, Virginia.

The encased heater 18 elevates the temperature of the humidification material 24 thereby elevating the temperature of the moisture it contains. The elevated temperature of the moisture leads to the creation of a vapor absorbed into the gas as it flows over the humidification material 24. Preferably, the humidification material 24 has a configuration that presents a high surface area to the direction of gas flow to allow increased opportunity for the moisture to evaporate into the gas thereby humidifying the gas.

In Fig. 2, turbulence of the gas is created by the interior of the shell 26 covering the humidification material 24 and heater 18 having a surface area that is of an irregular pattern or texture. This turbulence may be created using a variety of structures. These structures may be located, for example, on or as part of the shell 26 or humidification material 24. Further example of a structure for creating turbulence may be a spiral barrier. Other structures may be incorporated, for example, by being either attached to the humidification material 24 or interior of the shell 26 of the apparatus 1.

The moisture applied to the humidification material 24 can contain medications or additives that will evaporate and be carried along in the humidified gas to the patient. Levels of medication and/or fluid in the gas can be controlled by timed evaporation and adsorption rates. Fluid could be infused by syringe, gravity feed through tubing, or by any number of pumps, to retain proper saturation levels.

The apparatus 1 will have a port 16 for the infusion of fluid for the production of moisture. Moisture may include sterile water, medication, or a mixture of fluids required for merely humidification or dispensing of medication. The port 16 can be of the standard injection port used typically in the medical industry, a valve, or any other device, which can open or close allowing for the entrance of the fluid.

The apparatus 1 includes one or more temperature sensing devices (not shown) to regulate the heater 18. Each temperature-sensing device can be a resistive temperature device (RTD), a thermister, or a thermocouple. A K type thermocouple is embedded inside the heater 18 to measure its temperature. Any number of heater controller manufacturers such as Watlow or Hot Watt can provide the temperature sensing and control device. As shown in Figure 1, the shell 26 is an oblong tube having an internal channel, but any shape that will accommodate the internal elements of the device is acceptable. The internal channel of the shell 26 will be smooth. Any form of surface irregularity to promote turbulence without flow restriction is acceptable for the 15 internal channel of the shell 26. The shell 26 has an output opening 28 and an input opening 12 for the gas. The shell 26 additionally has a fluid fill port 16 for the infusion of fluid. Although, other methods of inserting the appropriate fluid or medicine in the shell 26 are possible.

Overall length will be between 3½ and 4 inches. Preferably, the apparatus 1 will weigh approximately four ounces. The shell 26 can be made of any suitable material, for example, metal or plastic.

In additional embodiments, as shown In Figure 3, a humidity sensor 34 may be included in the apparatus 1. Appropriate humidity sensors 34 can be obtained from Omega Corporation located in Atlanta, Georgia.

Optionally, in addition to the temperature sensing device described above, an external temperature sensing device 32 can be inserted in the insufflation tubing 10 just outside of the gas outlet 28. The same types of temperature sensing devices Internal to the apparatus 1 as described above can be used. This device 32 measures the downstream temperature of the gas.

The temperature of the gas is related to the temperature of the heater 18. The temperature sensing device located within the heater 18 measures the temperature of the heater 18. The temperature of the gas is not directly measured. Rather, the resulting temperature of the gas correlates to the temperature of the heater.

The warmed and humidified gas leaves the apparatus 1 through a gas outlet 28. The gas outlet may be a series of holes. The gas then enters the insufflation tubing 10 for possible delivery to a patient.

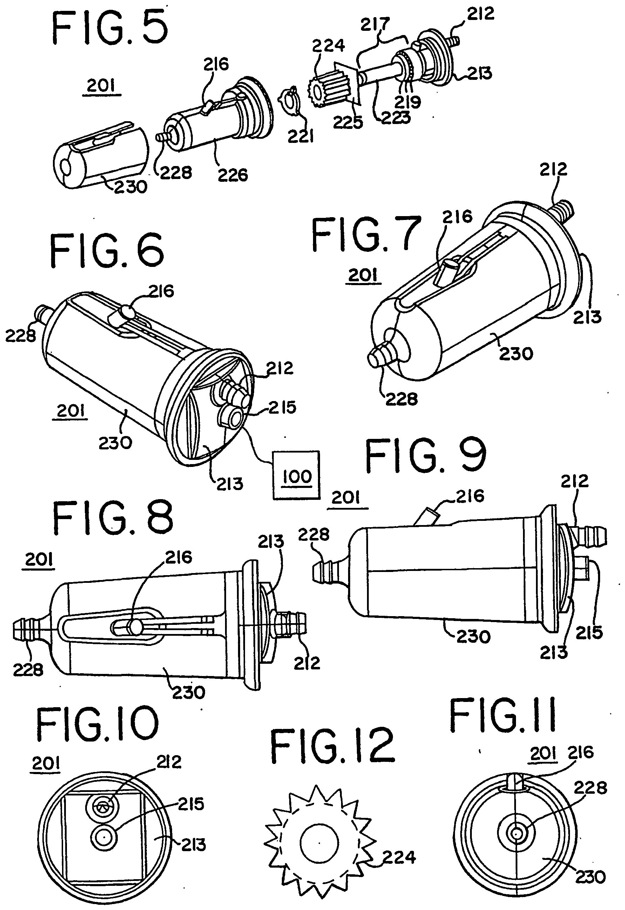

A first embodiment of a gas humidification apparatus is shown in Figs. 5-12. In a manner similar to the devices of Figs. 1-4, the gas humidification apparatus 201 can be used in conjunction with an insufflation device. In particular the gas humidification apparatus 201 is located downstream from a gas source for the insufflation device. The gas humidification apparatus 201 may be constructed as a re-useable or disposable product.

As shown in Figs. 5, 6, 9 and 10, a gas inlet port 212 is attached through a side portion of a front cap 213 of the gas humidification apparatus 201. In addition, an inlet port 215 is attached through a central portion of the front cap 213. The inlet port 215 allows for electrical components and wiring to be inserted into the gas humidification apparatus 201. The gas humidification apparatus 201 can be modified so that the ports 212 and 215 are interchanged with one another.

As shown in Fig. 5, the cap 213 includes an annular metallic heater housing 217 that is attached thereto. The heater housing 217 is in fluid communication with the gas inlet port 212. The heater housing 217 contains a heater cartridge that is well known in the art. When activated the heater cartridge heats up the interior and body of the heater housing 217 so that gases within and outside the heater housing 217 are heated. The heater housing 217 also includes a plurality of circular holes 219 having a diameter of approximately 0.1" (0.254cm). Other shapes and sizes for the holes 219 are possible, such as triangular and square shaped openings. When gas flows into the gas humidification apparatus 201 via the gas Inlet port 212, the gas flows into the heater housing 217, where it is heated if necessary, and then flows out of the holes 219. As shown in Fig. 5, there are approximately sixteen holes 219 that are arranged equidistantly from one another along an annular ring. The holes 219 of the heater housing 217 improve the rate of heating of the gas within the gas humidification apparatus 201 and create turbulence for the gas flowing within the gas humidification apparatus 201.

Two of the holes 219 preferably have their own RTD sensor. These sensors operate in the same manner as the temperature sensors for the examples of Figs. 1-4. In particular, the temperature measured by the two sensors are compared with one another to determine if one or both of the sensors is defective.

As shown in Fig. 5, a rear cylindrical portion 223 of the heater housing 217 is snugly inserted into a cylindrical central opening of a humidification material 224 that is preferably made of the same material as the humidification materials 24 described previously with respect to Figs. 1-4. A washer 221 is fitted over the rear portion 223 and abuts against the rear face of the humidification material 224 and acts as a stop in that it prevents the humidification material 224 from slipping off of the rear portion 223 and being wedged into an outlet 228.

In an alternative embodiment, the gas humidification apparatus 201 can further include a plate 225 positioned between the front or proximal end of the humidification material 224 and the heater housing 217. Since the holes 219 face the front end of the humidification material 224, the plate 225 allows the gas to flow along the exposed side of the humidification material 224. Note that the gas will flow along the side of the humidification material 224 with or without the presence of the plate 225.

As shown in Fig. 12, the humidification material 224 has a star-like pattern with ten to twelve points that aid in generating turbulence in the gas within the gas humidification apparatus 201 in a similar manner that the humidification material 24 of Figs. 1 and 4 do.

In an alternative embodiment, a second humidification material 224 may be spaced from the first humidification material by a spacer and out of phase with the first humidification material in the same manner as described previously with respect to the example of Figs. 1 and 4.

As shown in FIG. 5, the assembled humidification material 224 and washer 221 and the inlet port 215 and the heater housing 217 are inserted into a housing or shell 226. After insertion, the front cap 213 is screwed on or snap fit onto the heater housing 217. The housing 226 is made of a suitable material, such as plastic or metal, and has a downstream outlet 228 that allows the gas to flow outside of the housing 226.

As shown in FIGS. 5-9 and 11, the housing 226 includes a port 216 that allows fluid to be infused by syringe, gravity feed through tubing, or by any number of pumps, to the humidification material 224. The fluids infused may include sterile water, medication, or a mixture of fluids required for merely humidification or dispensing of medication. The interior end of the port 216 is positioned so that infused fluids drip into the housing 226 and are soaked up by the entire humidification material 224 by capillary action. The port 216 is similar to the port 16 described previously with respect to the examples of FIGS. 1-4.

As shown in FIGS. 5-9 , the housing 226 is inserted into a sleeve or shroud 230 so that the port 216 is slid along a slit 232 formed in the sleeve 230 and the outlet 228 extends through a rear opening 234 of the sleeve 230. The sleeve 230 is snap fit to the housing 226. The sleeve 230 is made of a thermal insulation material that retains the heat within the housing 226 so that a person can handle the sleeve 230 without fear of being exposed to excessive heat and without significantly heating up the ambient atmosphere.

Note that the sleeve 230, the housing 226 and the humidification material 224 may be disposable while the cap 213 and its attached heater housing 217 may be reusable.

The gas humidification apparatus 201 may include the temperature sensors, humidity sensors and control circuitry previously described with respect to the examples of FIGS. 1-4 and 18 so that the temperature and humidity of the gas flowing within the apparatus and delivered to a patient via outlet 228 is controlled,

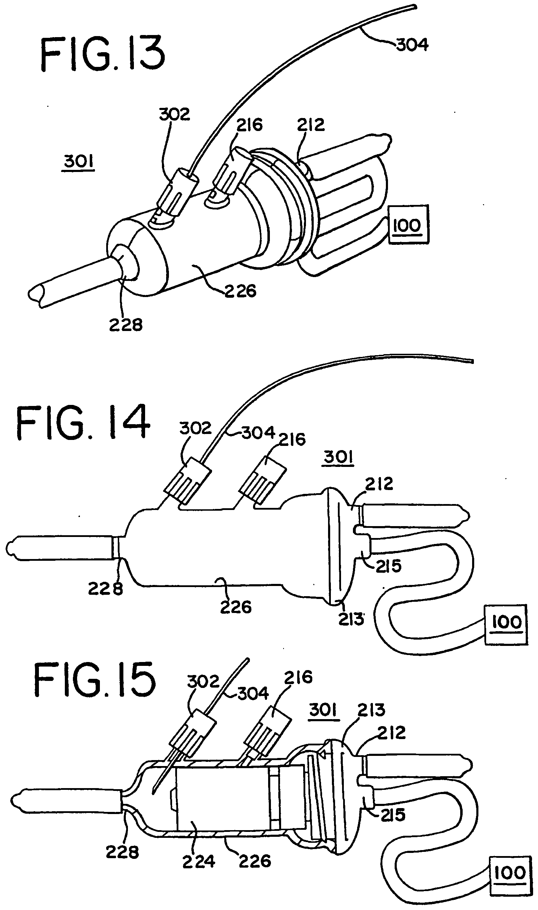

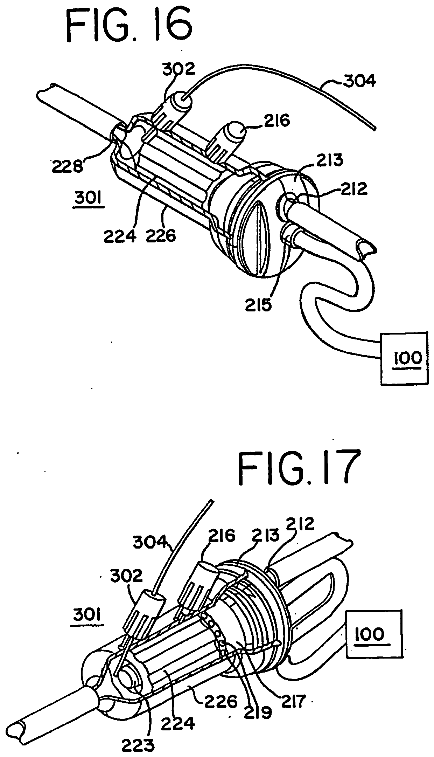

A second embodiment of a gas humidification apparatus is shown In Figs. 13-17. The gas humidification apparatus 301 essentially has the same structure as the gas humidification apparatus 201 of Figs. 5-12 and so like components will be designated with like numerals. One difference is that a second port 302 is added to the housing 226. The second port 302 is positioned between the humidification material 224 and the outlet 228 so as to allow a distal end of a catheter 304 to be inserted into the port 302. Depending on the intended material to be delivered to the patient, the distal end of the catheter 304 may be positioned within the port 302, within the interior of the gas humidification apparatus 301 or within a tube attached to the outlet 228 and in fluid communication with a section of a patient, or within the section of the patient. An example of a catheter that can be inserted into the gas humidification apparatus 201 is the catheter described in U.S. Patent No. 5,964,223. Other devices can be inserted into the port 302 in a similar manner as described above with respect to catheter 304, such as a lumen and an endoscope. Furthermore, gases, liquids, aerosols and medicines may be conveyed to a patient by a tube or other know dispensing devices inserted through the port 302 and exiting out of the outlet 228 into the patient. Note that the materials dispensed into the port 302 by the above-mentioned dispensing devices may have properties that raise the humidity of the gas within the interior of the gas humidification apparatus 301.

The gas humidification apparatus 301 may include the temperature sensors, humidity sensors and control circuitry previously described with respect to the embodiments of FIGS. 1-4 and 18 so that the temperature and humidity of the gas flowing within the apparatus and delivered to a patient is controlled.

In each of the devices for humidifying and/or warming a gas described previously with respect to FIGS. 1-18, it is desired that the flowing gas achieves a humidity that ranges from approximately 80 to 100 percent humidity and achieves a temperature that ranges from approximately 90 to 105 degrees Fahrenheit at a constant flow rate of approximately 20 liters per minute.

## Claims

1. A gas humidification apparatus (201) for use in a medical procedure comprising:
a housing (226) defining a channel having a gas inlet (212) and a gas outlet (228) in fluid communication with a humidification device;
said humidification device being in fluid communication with said inlet, said humidification device comprising:
a heater (18) for warming a gas flowing through the channel;
a humidification material (224) adjacent to said heater (18) that readily absorbs moisture and readily releases moisture when exposed to a dry environment;
an insufflator (10) comprising a gas source and connected to said humidification device receiving said gas from said insufflator which receives said gas from said gas source **characterized in that**
a control (100) is tied to said insufflator so as to control said heater and said insufflator by dynamically controlling temperature, gas volume, gas flow rate and humidity.

2. The apparatus of claim 1, wherein said humidification device has a star like pattern with 10 to 12 points for flowing gas over said humidification material.

3. The apparatus of claim 1, wherein said heater comprises a heater housing (217) in fluid communication with said gas inlet.

4. The apparatus of claim 3, wherein said heater housing comprises a plurality of holes (219).

5. The apparatus of claim 1, wherein said humidification material comprises a cylindrical central opening.

6. The apparatus of claim 5, wherein said heater comprises a rear cylindrical portion for insertion into the cylindrical central opening.

7. The apparatus of claim 4, wherein the plurality of holes (219) directs the gas such that it is allowed to flow over said humidification material.

8. The apparatus of claim 1, wherein said humidification device comprises a port added to the housing (226).

9. The apparatus of claim 8, wherein the port is adapted for a dispending device to be inserted within said port for delivering a medicament in aerosol form to humidified gas as it exits said gas outlet.

10. The apparatus of claim 1, said gas source is a source of carbon dioxide.

## Patentansprüche

1. Gasbefeuchtungsvorrichtung (201) zur Verwendung in einem medizinischen Verfahren, umfassend:
ein Gehäuse (226), das einen Kanal mit einem Gaseinlass (212) und einem Gasauslass (228) in Fluidverbindung mit einer Befeuchtungsvorrichtung definiert;
wobei die Befeuchtungsvorrichtung in Fluidverbindung mit dem Einlass steht, wobei die Befeuchtungsvorrichtung umfasst:
eine Heizvorrichtung (18) zum Erwärmen eines durch den Kanal strömenden Gases;
ein Befeuchtungsmaterial (224) angrenzend an die Heizvorrichtung (18), das leicht Feuchtigkeit absorbiert und leicht Feuchtigkeit abgibt, wenn es einer trockenen Umgebung ausgesetzt ist;
einen Insufflator (10), der eine Gasquelle umfasst und mit der Befeuchtungsvorrichtung verbunden ist, die das Gas von dem Insufflator empfängt, der das Gas von der Gasquelle empfängt;
**dadurch gekennzeichnet, dass**
eine Steuerung (100) mit dem Insufflator verbunden ist, um die Heizvorrichtung und den Insufflator durch dynamische Steuerung von Temperatur, Gasvolumen, Gasdurchflussrate und Feuchtigkeit zu steuern.

2. Vorrichtung nach Anspruch 1, wobei die Befeuchtungsvorrichtung ein sternförmiges Muster mit 10 bis 12 Punkten aufweist, um Gas über das Befeuchtungsmaterial strömen zu lassen.

3. Vorrichtung nach Anspruch 1, wobei die Heizvorrichtung ein Heizvorrichtungsgehäuse (217) in Fluidverbindung mit dem Gaseinlass umfasst.

4. Vorrichtung nach Anspruch 3, wobei das Heizvorrichtungsgehäuse eine Vielzahl von Löchern (219) aufweist.

5. Vorrichtung nach Anspruch 1, wobei das Befeuchtungsmaterial eine zylindrische Mittelöffnung aufweist.

6. Vorrichtung nach Anspruch 5, wobei die Heizvorrichtung einen hinteren zylindrischen Abschnitt zum Einführen in die zylindrische Mittelöffnung aufweist.

7. Vorrichtung nach Anspruch 4, bei der die Vielzahl von Löchern (219) das Gas so leitet, dass es über das Befeuchtungsmaterial strömen kann.

8. Vorrichtung nach Anspruch 1, wobei die Befeuchtungsvorrichtung eine an das Gehäuse angebrachte Öffnung (226) aufweist.

9. Vorrichtung nach Anspruch 8, wobei die Öffnung für eine Abgabevorrichtung angepasst ist, die in die Öffnung eingesetzt wird, um ein Medikament in Aerosolform an befeuchtetes Gas abzugeben, wenn es den Gasauslass verlässt.

10. Vorrichtung nach Anspruch 1, wobei die Gasquelle eine Kohlendioxidquelle ist.

## Revendications

1. Appareil d'humidification de gaz (201) pour l'utilisation dans une procédure médicale comprenant :
un logement (226) définissant un canal ayant un orifice d'entrée de gaz (212) et un orifice de sortie de gaz (228) en communication fluidique avec un dispositif d'humidification ;
ledit dispositif d'humidification se trouvant en communication fluidique avec ledit orifice d'entrée, ledit dispositif d'humidification comprenant :
un dispositif de chauffage (18) pour chauffer un gaz s'écoulant à travers le canal ;
un matériau d'humidification (224) adjacent audit dispositif de chauffage (18) qui absorbe facilement l'humidité et libère facilement l'humidité lorsqu'il est exposé à un environnement sec ;
un insufflateur (10) comprenant une source de gaz et relié audit dispositif d'humidification recevant ledit gaz dudit insufflateur qui reçoit ledit gaz de ladite source de gaz
**caractérisé en ce que**
un dispositif de commande (100) est solidement fixé audit insufflateur afin de commander ledit dispositif de chauffage et ledit insufflateur en régulant dynamiquement la température, le volume de gaz, le débit d'écoulement du gaz et l'humidité.

2. Appareil selon la revendication 1, ledit dispositif d'humidification ayant un motif en étoile avec 10 à 12 points d'écoulement de gaz sur ledit matériau d'humidification.

3. Appareil selon la revendication 1, ledit dispositif de chauffage comprenant un logement de dispositif de chauffage (217) en communication fluidique avec ledit orifice d'entrée de gaz.

4. Appareil selon la revendication 3, ledit logement de dispositif de chauffage comprenant une pluralité de trous (219).

5. Appareil selon la revendication 1, ledit matériau d'humidification comprenant une ouverture centrale cylindrique.

6. Appareil selon la revendication 5, ledit dispositif de chauffage comprenant une portion cylindrique arrière pour l'insertion dans l'ouverture centrale cylindrique.

7. Appareil selon la revendication 4, la pluralité des trous (219) dirigeant le gaz de sorte qu'il est laissé à s'écouler par-dessus ledit matériau d'humidification.

8. Appareil selon la revendication 1, ledit dispositif d'humidification comprenant un orifice ajouté au logement (226).

9. Appareil selon la revendication 8, l'orifice étant adapté pour qu'un dispositif de distribution soit inséré à l'intérieur dudit orifice pour l'administration d'un médicament sous forme d'aérosol vers le gaz humidifié lorsqu'il sort dudit orifice de sortie de gaz.

10. Appareil selon la revendication 1, ladite source de gaz étant une source de dioxyde de carbone.
